# EUROPEAN PATENT APPLICATION

(11) **EP 3 258 267 A1**
(43) Date of publication of application: **20.12.2017**
(21) Application number: 16174169.9
(22) Date of filing: 13.06.2016
(51) Int. Cl.: G01N 33/543, G01N 33/68

(54) **METHOD TO DETECT INFECTIONS AND SEROLOGICAL STATUS OF HUMANS OR ANIMALS**

(71) Applicant: Siemens Healthcare GmbH, 91052 Erlangen (DE)
(72) Inventor: Kappel, Andreas, 61479 Glashütten (DE); Matzas, Mark, 91336 Heroldsbach (DE)

(57) **Abstract**

The present invention relates to a method of detecting a plurality of antibodies, including determination of the class of these antibodies, in a sample of a patient, and a kit that can be used in such a method.

## Description

The present invention relates to a method of detecting a plurality of antibodies, including determination of the class of these antibodies, in a sample of a patient, and a kit that can be used in such a method.

Infections by bacteria, viruses or parasites have always been a threat to humans, and will continue to count for a high number of deaths in the world. The most widely used strategies to fight infections are
a) prevention (e.g. by vaccination) or
b) treatment (e.g. by antibiotics or antiviral agents).

For both these strategies, diagnostic methods that detect ongoing infections (for treatment) or previous infections (to check if immunity by a previous infection or a vaccination is provided) are needed. Many if not most of these methods are so-called indirect tests where not the infectious agent but rather the immune response against it is measured. For example, the well-known HIV or Hepatitis tests do not necessarily detect the presence of the viruses in the blood of an individual, but rather detect if antibodies against the virus are present.

Antibodies against infectious agents have the advantage that they can be easily detected by standard laboratory methods. Moreover, their presence indicates if immunity is given, which has an impact on medical decisions for or against vaccination. Finally, the measurement of certain antibodies can indicate the stage of the infection, and can also help to discriminate between acute and chronic infections.

Antibodies against infectious agents - in particular those of the IgG class - often remain present in an individual's blood long time after the infection has happened, indicating that the individual has been exposed to the agent, and thereby providing guidance for treatment or vaccination. In particular, if the titer (i.e. the concentration of the antibody in the blood) drops between a certain threshold, a vaccination is indicated. It is important to note that current infections can often be discriminated from previous infections by measuring IgM instead of IgG, as IgM towards an infectious agent is often only present during acute infection, whereas IgG levels remain often high even if the infection has long been resolved.

Current serological methods to detect viral infections are predominantly limited to testing one pathogen at a time and are therefore used primarily to address specific clinical hypotheses. A method that could simultaneously detect responses to all human pathogens, particularly e.g. viruses, would allow hypothesis-free analysis to detect associations between past viral infections and particular diseases or population structures.

However, only one method has recently been described that could provide these features.

Elledge et al. (WO2015/095355), and also GJ Xu et al. (Science 05 Jun 2015: Vol. 348, Issue 6239, pp: "Comprehensive serological profiling of human populations using a synthetic human virome") have recently described a method that can detect antibodies to all known viruses in a single test with a human blood sample, therefore providing information if this individual has ever been or currently is exposed to one or more of these viruses.

In this method, viral proteins are individually expressed in bacteriophages that can be propagated by growing them in bacteria. A library of bacteriophages (phage) is then generated where each bacteriophage contains a defined viral protein. In a next step, a human plasma, serum or whole blood sample is incubated with the bacteriophage library. If antibodies against viral proteins are present in the sample, these antibodies would bind to respective bacteriophages and precipitate them ("Immunoprecipitation"). The precipitated phages can be purified by several centrifugation and washing steps. Next, the DNA of the phages can be amplified by PCR (polymerase chain reaction) and sequenced, thus allowing to detect for which viral proteins the precipitated phages code for. Thereby, the phages/viral proteins to which antibodies were present in the sample can be traced back.

Although this method clearly addresses an important clinical need, it has several drawbacks:
- It is challenging to establish a GMP (good manufacturing practice)-compliant manufacturing process for the phage library: Several sub-libraries with an extremely well defined cfu (colony forming unit) count and shelf life stability need to be manufactured and then blended to create the actual library. The effort for QC (quality control) testing may be enormous.
- Immunoprecipitation is not necessarily a sensitive method, which may make it difficult to detect certain low-affinity antibodies. While this may be overcome by making the subsequent PCR amplification more sensitive, this again may lead to more false positive reactions since the washing steps may not completely remove unbound phages from the immune precipitate. In summary, the tradeoff between sensitivity and specificity of the test may be difficult to establish.
- The method is built up from quite expensive sub-steps (Immunoprecipitation, Phage library manufacturing, Sequencing), which may make the test more expensive than the estimate of 25 US$ given before, in particular when reagents need to be manufactured in a GMP environment.
- The test may be impractical for clinical routine, as it is composed of several manual steps that cannot be integrated and automated.
- The method can hardly distinguish between acute and recent infection
- The phages encode for protein fragments that may lack both three-dimensional conformation of the native antigen. Moreover, those protein fragments may lack the posttranslational modifications of the natural antigen, as they are produced in bacteria and not eukaryotic cells. As many antibodies need properly folded antigens with the correct posttranslational modification in order to bind, the phage-based method may not be able to detect certain antibodies to the respective antigen, which may lead to false negative test results.

In order to overcome these drawbacks, the inventors propose an improved method that addresses the same clinical need as the method described by Elledge et al., which lacks the limitations thereof.

### Summary of the invention

The present inventors found that a fast and cheap detection of a plurality of antibodies as well as determination of the class thereof can be obtained when these are reacted with a plurality of immobilized antigens and thereafter the antibodies are further reacted with markers for IgM, IgG and/or IgA.

In a first aspect, the present invention relates to a method of detecting a plurality of antibodies, including determination of the class of these antibodies, in a sample of a patient, comprising the steps of:
a) providing a plurality of coded beads on which different antigens are immobilized, wherein on each different kind of coded bead a different antigen or antigen mixture is immobilized, and wherein the codes of the beads can be detected and discriminated by a first detection method;
b) bringing the plurality of coded beads in contact with the sample;
c) reacting the plurality of coded beads with the sample;
d) adding differently labelled markers for at least two of IgM, IgG and/or IgA to the sample reacted with the coded beads, and reacting the labelled markers with the coded beads reacted in step c), wherein the labels for IgM, IgG and/or IgA can be detected and discriminated by a second detection method different to the first detection method; and
e) detecting the code of the beads in combination with labels of the markers for IgM, IgG and/or IgA to determine which code of the reacted beads is associated with which marker of IgM, IgG and/or IgA.

In a further aspect the invention relates to a kit, comprising a plurality of coded beads on which different antigens are immobilized, wherein on each different kind of coded bead a different antigen or antigen mixture is immobilized, and wherein the codes of the beads can be detected and discriminated by a first detection method; and differently labelled markers for at least two of IgM, IgG and/or IgA, wherein the labels of the markers for IgM, IgG and/or IgA can be detected and discriminated by a second detection method different to the first detection method.

Further aspects and embodiments of the invention are disclosed in the dependent claims and can be taken from the following description and examples, without being limited thereto.

### Detailed description of the invention

### Definitions

Unless defined otherwise, technical and scientific terms used herein have the same meaning as commonly understood by one of ordinary skill in the art to which this invention belongs.

In the context of the present invention a "sample" is a sample which comprises potentially at least a plurality of antibodies to be detected. Examples for samples are: cells, tissue, and/or biopsy specimens that are brought into a form to be reacted with a plurality of coded beads on which different antigens are immobilized, e.g. by dissolution, etc.; body fluids such as blood, urine, saliva, sputum, plasma, serum, cell culture supernatant, swab sample and others. According to certain embodiments, the sample is a patient sample (clinical isolate). Preferred samples are serum, plasma, and/or whole blood of a patient.

According to certain embodiments, the patient in the present methods is a vertebrate, more preferably a mammal and most preferred a human patient.

A vertebrate within the present invention refers to animals having a vertebrae, which includes mammals - including humans, birds, reptiles, amphibians and fishes. The present invention thus is not only suitable for human medicine, but also for veterinary medicine.

Antibodies, also known as immunoglobulins, are not particularly limited in the present invention and can be derived from a variety of antigen reactions in the body. They can be produced by the body of the patient in reaction to an external stimulus, particularly a pathogen, e.g. a microorganism like virus, bacteria, protozoa, parasites and/or pathogenic nematodes, which act as antigens to which the antibodies are produced by the patient. According to certain embodiments, the antibodies to be detected were produced by the patient in reaction to one or more pathogens, particularly viruses and/or nematodes. A pathogen within the present invention is anything that can cause a disease in a patient, particularly an infection agent such as a virus, bacterium, prion, fungus, nematode, or another microorganism.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

In a first aspect, the present invention relates to a method of detecting a plurality of antibodies, including determination of the class of these antibodies, in a sample of a patient, comprising the steps of:
a) providing a plurality of coded beads on which different antigens are immobilized, wherein on each different kind of coded bead a different antigen or antigen mixture is immobilized, and wherein the codes of the beads can be detected and discriminated by a first detection method;
b) bringing the plurality of coded beads in contact with the sample;
c) reacting the plurality of coded beads with the sample;
d) adding differently labelled markers for at least two of IgM, IgG and/or IgA to the sample reacted with the coded beads, and reacting the labelled markers with the coded beads reacted in step c), wherein the labels for IgM, IgG and/or IgA can be detected and discriminated by a second detection method different to the first detection method; and
e) detecting the code of the beads in combination with labels of the markers for IgM, IgG and/or IgA to determine which code of the reacted beads is associated with which marker of IgM, IgG and/or IgA.

According to certain embodiments, at least differently labelled markers for IgM and IgG are added in step d), further preferably differently labelled markers for IgM, IgG and/or IgA.

Step a) is not particularly limited and can be carried out suitably, e.g. by already obtaining coded beads with immobilized antigens, or by reacting different coded beads with different antigens or antigen mixtures. Is should be ensured that the antigens or antigen mixtures immobilized on differently coded beads differ and are known for each type of coded bead.

Step b) also can be suitably carried out without restriction, and can be done by mixing the coded beads with the sample, e.g. in reaction tubes, a microfluidic system, wells of a microtiter plate, etc. According to certain aspects, it is also possible to keep differently coded beads in different reaction vessels and add aliquots of the sample to each, as e.g. in immunoassays, but the sample can be added to the mixture of coded beads as well as a distinction of the different antibodies can be done by the second reaction step with the labelled markers for IgM, IgG and/or IgA. If the amounts of coded beads are known for each bead, respectively, as well as the amount of immobilized antigens, a quantification of the detected antibodies in the sample is possible. The quantification can be then carried out in a usual way, taking into account the number of detected coded beads for which also a signal is obtained from the labelled markers. For step b), the coded beads with the immobilized antigens and/or antigen mixture can be also dispersed in a suitable solvent or solvent mixture or solvent system, facilitating e.g. mixing with the sample. The solvent/solvent system can contain additives that facilitate mixing as long as they do not affect the reaction in step c).

Step c) can be carried out in a suitable way, taking into account the reaction between antigens and antibodies for each antibody to be detected. Temperature and reaction time can be suitably set, depending on the different antigens used.

In step d) the adding of differently labelled markers for IgM, IgG and/or IgA to the sample reacted with the coded beads, and reacting the labelled markers with the coded beads reacted in step c) is also not particularly limited, and adding can be e.g. carried out in a similar fashion as in step b), e.g. in a microfluidic system by adding a further flow channel or adding the labelled markers through the same channel. The differently labelled markers can be added in a suitable solvent or solvent mixture or solvent system that preferably does not negatively affect the beads, the immobilized antigens and the reacted antibodies, which can be suitably selected. The solvent/solvent system can contain additives that facilitate mixing as long as they do not affect the reaction in step c).

According to certain embodiments, also a washing step with a suitable washing liquid can be carried out prior to adding the differently labelled markers for IgM, IgG and/or IgA to the sample, so that non-reacted (and thus not immobilized) components of the sample are washed out. According to certain embodiments, only reacted antibodies of the samples stay on the coded beads, so that in a washing step only those remain from the sample. Again, the reaction with the labelled markers can be suitably carried out, taking into account e.g. the marker types and the antibodies. Also after the reaction with the labelled markers a washing step can be carried out to remove unreacted labelled markers. However, as the signals for the coded beads and the labelled markers are correlated only in case an antibody has reacted with them, and as both signals can be obtained at essentially the same time according to certain embodiments, also this correlation can be taken to filter out signals due to unreacted markers and/or coded beads.

Further, also step e) is not particularly limited, and suitable detection methods can be selected based on the code of the beads and the label of the markers.

In the present invention, the beads are not particularly restricted as long as antigens can be immobilized thereon. They can be surface-treated to facilitate immobilization of the antigens. According to certain embodiments, the beads are microparticles, particularly with a diameter of between 1 and 1000 µm, preferably between 10 and 700 µm, further preferably between 50 and 500 µm, as they allow for sufficient amounts of antigens to be immobilized while they still can be easily detected. Also, microparticles can be easily detected in a microfluidic system, respectively microfluidic device, thus facilitating automation of the present method,

IgM, IgG and IgA in the present invention represent immunoglobulin M, immunoglobulin G and immunoglobulin A, respectively. Due to the detection of either of those it is possible to discriminate the class of antibody, which in turn can be used for determining whether the antibody was produced as a reaction to a prior external stimulus, e.g. a pathogen, or a recent, acute external stimulus like a pathogen. In this instance, normally IgG antibodies tend to decrease in level generally slower than IgM antibodies, so that a stronger signal for IgG can most likely be correlated to a recent or ongoing infection or vaccination, etc. whereas higher levels of IgM are more likely related to an acute infection. The reaction and presence of the different antibodies can thereby vary between different external stimuli, e.g. between particular pathogens, e.g. viruses and/or nematodes, etc. IgA can also be used to detect acute and previous infections, and it has a high titer especially in sputum. As the reactions of these are known, a determination of a current infection, etc. or a titer due to a previous infection, vaccination, etc. can normally be discriminated.

The different antigens or mixtures of antigens immobilized in step a) are not particularly limited and can be suitably obtained. In case of detection of antibodies against certain pathogens, these antigens can be e.g. directly obtained from the pathogens themselves, e.g. by digesting them. According to certain embodiments, the antigens are from pathogens and are surface antigens.

The antigens can then be further purified by known methods. Alternatively, also recombinant antigens can be obtained using known methods, or they can be obtained as synthetic peptides. According to certain embodiments, the antigens are native and/or recombinant antigens and/or synthetic peptides. According to certain embodiments, the antigens are native antigens. The antibodies in the sample can be directed to different epitopes and/or antigens. Using a mixture of antigens, preferably native antigens, thus allows a thorough detection of the antibodies, e.g. detecting them also in case they are only detected to native antigens and not specific epitopes. Using the above bacteriophage approach, usually only certain epitopes are addressed, so that there is a risk that not all antibodies are detected.

For different kinds of external stimuli, e.g. pathogens, different antigens can be considered. A selection of antigen sources in combination with immunoglobulin classes to be tested is shown in Table 1.

**Table 1: Preferred antigens and immunoglobulin classes to be tested for specific pathogens**

| Pathogen | Preferred Immunoglobulin classes to be tested | Preferred Antigen(s) |
|---|---|---|
| Adenovirus | IgG/IgM/IgA | purified or recombinant antigens of Adenovirus |
| Ascaris lumbricoides | gG/IgM/IgA | purified or recombinant antigens of Ascaris lumbricoides |
| Bordetella pertussis | IgG/IgM/IgA | • IgA, IgG: B. pertussis Toxin and filamentous Hemagglutinine. |
| | | • IgM Purified lysate antigens of B. pertussis |
| Borrelia burgdorferi | IgG/IgM | • IgG: OspC (B. sensu stricto, B. garinii), p100 (B. afzelii), p18 (B. afzelii), p41i (B. garinii), VlsE (recombinant) IgM: OspC (B. afzelii, B. garinii), p41i (B. garinii) |
| Brucella | IgG/IgM | Purified or recombinant Brucella abortus (W 99) antigens |
| Chikungunya virus | IgG/IgM | Purified or recombinant Chikungunya antigens |
| Clamydia trachomatis | IgG/IgM/IgA | Purified or recombinant C. trachomatis LGV Type II strain 434 antigens |
| Clamydia pneumoniae | IgG/IgM/IgA | purified or recombinant C. pneumoniae CWL-29 antigens |
| Clostridium tetani | IgG/IgM | Tetanus Toxoid antigens |
| Corynebacterium diphtheriae | IgG/IgM | Corynebacterium diphtheriae Toxoid antigens |
| Coxiella burnetii (Q-Fever) | IgG/IgM | Purified or recombinant Coxiella burnetii IgG antigens |
| Cytomegalovirus (CMV) | IgG/IgM | • IgG: CMV strain AD-169 antigens |
| | | • IgM: CMV strain Davis antigens |
| Dengue virus | IgG/IgM | Purified or recombinant Dengue Virus 2 antigens e.g. from strain 16681 |
| Echinococcus granulosus or multiocularis | IgG/IgM | purified or recombinant antigens of Echinococcus |
| Entamoeba histolytica | IgG/IgM | Purified or recombinant Entamoeba histolytica Trophozoit antigens |
| Epstein Barr Virus (EBV) | IgG/IgM | p18 peptide of the Epstein-Barr virus |
| Helicobacter pylori | IgG/IgA | • IgA: recombinant CagA- and recombinant urease-antigens |
| | | • IgG: Highly purified proteins associated with CagA genes (120 KD) and VaCA genes (87 KD) as well as urease-antigens |
| Hepatitis A virus (HAV) | IgG/IgM | Purified hepatitis A virus protein |
| Hepatitis B virus (HBV) | IgG/IgM | Individual tests with beads coated with HBc, HbsAg, HBe |
| Hepatitis C virus (HCV) | IgG/IgM | Recombinant or purified peptides from HCV such as C22 |
| Hepatitis D virus (HDV) | IgG/IgM | Purified or recombinant hepatitis D virus protein |
| Hepatitis E virus (HEV) | IgG/IgM | Recombinant or purified ORF 2 and 3 peptides from HEV, Burma, Mexican or Pakistan strain |
| Herpes Simplex Virus (HSV) | IgG/IgM | • Recombinant gG1 proteins of HSV Type 1 and 2 |
| | | • purified membrane antigens of strain Mac Intyre (ATCC VR-539) (HSV1) and strain Ms (ATCC VR-540) (HSV2) |
| Human Immune-deficiency virus (HIV), type 1 and 2 | IgG/IgM | • HIV 1 gp41, |
| | | • HIV 1 (subtype O) gp41, |
| | | • HIV 2 gp36 |
| Influenza A virus | IgG/IgM/IgA | Purified or recombinant Influenza A grade 2 antigens (strain Texas 1/77 (H3N2)) |
| Influenza B | IgG/IgM/IgA | Purified or recombinant Influenza B grade 2 antigens (strain Hong Kong 5/72) |
| Legionella pneumophila | IgG/IgM | Legionella pneumophila antigens (serovars 1-7) |
| Leptospira | IgG/IgM | Leptospira biflexa antigens |
| Leishmania (major, donovani, infantum, mexicana, brasiliensis) | IgG/IgM | Purified or recombinant Leishmania antigens |
| Measles | IgG/IgM | Purified or recombinant measles virus antigens |
| Mumps virus | IgG/IgM | Purified or recombinant Parotitis (Mumps) Virus antigens |
| Parainfluenza virus (tpe 1-3) | IgG/IgA | Purified or recombinant Parainfluenza virus antigens |
| Parvovirus B19 | IgG/IgM | Recombinant or recombinant Parvovirus B19 antigens |
| Plasmodium (falciparum, vivax) | IgG/IgM | Recombinant CSP and MSP1 proteins from *Plasmodium vivax* and *Plasmodium falciparum* |
| Respiratory syncytial Virus (RSV) | IgG/IgM/IgA | Purified or recombinant Respiratory syncytial Virus antigens |
| Rubella virus | IgG/IgM | Purified or recombinant rubella antigens |
| Schistosoma mansonii | IgG/IgM | Purified or recombinant Schistosoma mansonii antigens |
| Taenia solium | IgG/IgM | Purified or recombinant Taenia solium antigens |
| Tickborne Encephalitis virus (TBE) | IgG/IgM | Purified or recombinant TBE (Tick Borne Encephalitis) antigens |
| Toxoplasma gondii | IgG/IgM | • Toxoplasma gondii lysate (IgG) |
| | | • Toxoplasma gondii chimeric antigens (IgM) |
| Toxocara canis | IgG/IgM | Purified or recombinant Toxocara canis antigens |
| Treponema pallidum | IgG/IgM | Recombinant antigens r15, r17 and r44, 5 |
| Trichinella spiralis | IgG/IgM | Purified or recombinant Trichinella spiralis antigens |
| Trypanosoma cruzi | IgG/IgM | Recombinant Trypanosoma cruzi antigens (TcF) |
| Varicella Zoster Virus (VZV) | IgG/IgM | Purified or recombinant Varicella-Zoster-Virus antigens |
| West Nile virus | IgG/IgM | Purified or recombinant West Nile virus antigens |

The coding of the beads is not particularly limited as long as it can be detected by a first detection method, including e.g. optical, electromagnetic, radiological and/or gravimetric methods. Optical detection can e.g. be warranted by differently colored beads or beads with different excitation spectra upon absorption. Radiological methods can e.g. include using different radioactive isotopes, wherein gravimetric methods can be based on beads with different weights and detection thereof, e.g. by cross field flow fractionation. Electromagnetic methods can be e.g. based on beads with different electric and/or magnetic properties. Also combination of different "codes" on the beads can be used. According to certain embodiments, the coded beads are color-coded, as this can be easily detected and the detection can be automated. Also, continuous detection is possible, like e.g. in microfluidic devices. The colors are not limited and can also encompass "colors" that are not encompassed within the visible range, e.g. UV- and/or IR-coded beads, as long as the colors can be detected and discriminated, e.g. using beads with absorption of electromagnetic radiation in a different wavelength spectrum.

The differently labelled markers for IgM, IgG and/or IgA are not particularly limited and can be any markers that are suitably labeled. Makers include antibodies for IgM, IgG and/or IgA as well as binding molecules that bind these specific immunoglobulins, e.g. aptamers, affibodies, nanobodies (also called single-domain antibodies), etc. According to certain embodiments the differently labelled markers for IgM, IgG and/or IgA are labelled antibodies and/or labelled binding molecules chosen from aptamers, affibodies, and/or nanobodies, e.g. labelled antibodies, as these can be easily obtained.

The labels for the differently labelled markers for IgM, IgA and IgG are also not particularly limited as long as they can be detected by a second detection method that can discriminate the signal from the one of the first detection method, meaning that preferably the detection principles in the first and second detection method should differ. According to certain embodiments, the differently labelled markers for IgM, IgG and/or IgA are fluorescence-labelled, i.e. can be detected by a fluorescence signal that is obtained upon excitation of the labels. In combination with color-coded beads this results in the further advantage that a irradiation source like a light source for detecting the color, e.g. due to absorption in the sample, can at the same time be used as an excitation source for the fluorescence label of the markers for IgM, IgA and IgG, thus allowing a fast and easy detection of both the code of the bead as well as the label of the markers for IgM, IgA and IgG. The labels can be suitably applied to the markers using known methods.

According to certain embodiments, the detection in step e) is carried out using a microfluidic system. This allows for an easy detection that can be automated. Further, microfluidic systems can be used for a simple quantification of signals, particularly for optical detection methods, as usually irradiation sources in these optical methods, like e.g. lasers, diodes, etc., can be chosen in such a way that they irradiate a whole microfluidic channel in a microfluidic system for detection, e.g. even essentially homogeneous. Also, washing steps can easily be integrated using a microfluidic system.

In certain embodiments the coded beads are magnetic. Such a magnetic property can be present in addition to the code used for the beads, e.g. a color code. The use of magnetic beads enables a further possibility to manipulate the beads during the method, e.g. allowing to keep the beads at a certain location during the method, e.g. for the reaction in step c) to allow a sufficient reaction time, and/or for an optional washing of the beads at any time in the method, e.g. after the reaction in step c) and/or step d) to allow washing off of non-bonded antibodies and/or markers to decrease a detection signal due to unspecific binding.

According to certain embodiments, the sample is chosen from serum, plasma and whole blood from the patient.

In a further aspect the present invention relates to a kit, comprising,
- a plurality of coded beads on which different antigens are immobilized, wherein on each different kind of coded bead a different antigen or antigen mixture is immobilized, and wherein the codes of the beads can be detected and discriminated by a first detection method;
- differently labelled markers for at least two of IgM, IgG and/or IgA, wherein the labels of the markers for IgM, IgG and/or IgA can be detected and discriminated by a second detection method different to the first detection method.
According to certain embodiments, the present kit can be used in the present method. The coded beads, antigens, labelled markers, etc. are not particularly limited and can be the same as laid out above with regard to the present method. Also, the first and detection method are not particularly restricted and can be as e.g. described above for detecting the code of the beads and the labels of the labelled markers, respectively.

According to certain embodiments, the coded beads are color-coded. According to certain embodiments, the differently labelled markers for IgM, IgG and/or IgA are labelled antibodies and/or labelled binding molecules chosen from aptamers, affibodies, and/or nanobodies. According to certain embodiments, the differently labelled markers for IgM, IgG and/or IgA are fluorescence-labelled. According to certain embodiments, the coded beads are magnetic. According to certain embodiments, the antigens are native and/or recombinant antigens and/or synthetic peptides.

### Examples

The present invention will now be described in detail with reference to several examples thereof. However, these examples are illustrative and do not limit the scope of the invention.

An exemplary method of the present invention is composed of the following steps:
1. Microparticles with a defined colour are coated with surface antigen(s) from defined infectious agent(s). The beads can also be magnetic, which allows to implement washing steps at any time of the procedure.
2. Microparticles coated with antigens of different infectious agents (thus having different colors) are blended to make a library that represents different infectious agents that can be identified by the individual color of the microparticle they are coated on.
3. The microparticle is incubated with a serum/plasma/whole blood sample from a patient, e.g. a human or animal. Antibodies to infectious agents present in the sample will bind to the respective beads.
4. The reaction product is subsequently incubated with fluorescence-labelled binders towards IgG or IgM. Those binders are typically monoclonal or polyclonal antibodies to IgG or IgM (e.g. anti-human IgG), and they can be differently labelled to discriminate IgG from IgM.
5. The thus-obtained reaction product is then passed through a microfluidic system for detection of the color of the beads and the fluorescence labels. Multiple light sources inside the system excite (1) the internal bead dyes that identify each microparticle and (2) fluorescent reporter binders (anti-IgG or IgM) captured during the assay. A detection instrument records readings for each bead set (e.g. absorption by beads and fluorescence from labels) and produces a distinct result for each analyte in the sample, i.e. it plots for how many beads with a certain dye how much fluorescence from anti-IgG or anti-IgM is generated.

Thereby, the method allows detecting how much IgG or IgM towards a certain infectious agent is present in a plasma/serum/whole blood sample.

Uncoated beads with different dyes as well as corresponding detection instruments can e.g. be purchased from Luminex.

The present invention enable the translation of the bacteriophage-based method described by Elledge et al into an immunoassay-based technology that can be performed on existing equipment in the clinical laboratory.

In contrast to the method described by Elledge et al.,
- the present method does not require a challenging GMP-compliant manufacturing process but rather uses established manufacturing procedures
- the present method may likely exhibit a higher sensitivity and specificity
- the present method is cheaper
- the present method can easily be automated and does not require labor-intensive steps

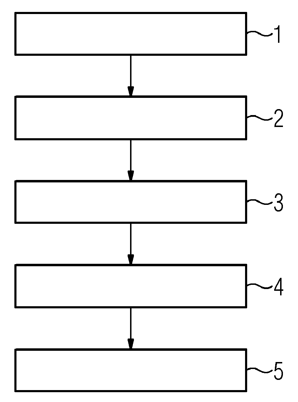

## Claims

1. A method of detecting a plurality of antibodies, including determination of the class of these antibodies, in a sample of a patient, comprising the steps of:
a) providing a plurality of coded beads on which different antigens are immobilized, wherein on each different kind of coded bead a different antigen or antigen mixture is immobilized, and wherein the codes of the beads can be detected and discriminated by a first detection method;
b) bringing the plurality of coded beads in contact with the sample;
c) reacting the plurality of coded beads with the sample;
d) adding differently labelled markers for at least two of IgM, IgG and/or IgA to the sample reacted with the coded beads, and reacting the labelled markers with the coded beads reacted in step c), wherein the labels for IgM, IgG and/or IgA can be detected and discriminated by a second detection method different to the first detection method; and
e) detecting the code of the beads in combination with labels of the markers for IgM, IgG and/or IgA to determine which code of the reacted beads is associated with which marker of IgM, IgG and/or IgA.

2. The method of claim 1, wherein the coded beads are color-coded.

3. The method of claim 1 or 2, wherein the differently labelled markers for IgM, IgG and/or IgA are labelled antibodies and/or labelled binding molecules chosen from aptamers, affibodies, and/or nanobodies.

4. The method of any one of the preceding claims, wherein the differently labelled markers for IgM, IgG and/or IgA are fluorescence-labelled.

5. The method of any one of the preceding claims, wherein the detection in step e) is carried out using a microfluidic system.

6. The method of any one of the preceding claims, wherein the coded beads are magnetic.

7. The method of any one of the preceding claims, wherein the sample is chosen from serum, plasma and whole blood from the patient.

8. The method of any one of the preceding claims, wherein the antigens are native and/or recombinant antigens and/or synthetic peptides.

9. A kit, comprising,
- a plurality of coded beads on which different antigens are immobilized, wherein on each different kind of coded bead a different antigen or antigen mixture is immobilized, and wherein the codes of the beads can be detected and discriminated by a first detection method;
- differently labelled markers for at least two of IgM, IgG and/or IgA, wherein the labels of the markers for IgM, IgG and/or IgA can be detected and discriminated by a second detection method different to the first detection method.

10. The kit of claim 9, wherein the coded beads are color-coded.

11. The kit of claim 9 or 10, wherein the differently labelled markers for IgM, IgG and/or IgA are labelled antibodies and/or labelled binding molecules chosen from aptamers, affibodies, and/or nanobodies.

12. The kit of any one of claims 9 to 11, wherein the differently labelled markers for IgM, IgG and/or IgA are fluorescence-labelled.

13. The kit of any one of claims 9 to 12, wherein the coded beads are magnetic.

14. The kit of any one of claims 9 to 13, wherein the antigens are native and/or recombinant antigens and/or synthetic peptides.
